# FASCICULE DE BREVET EUROPEEN

(11) **EP 2 387 974 B1**
(45) Date de publication et mention de la délivrance du brevet: **01.05.2013**
(21) Numéro de dépôt: 11166390.2
(22) Date de dépôt: 17.05.2011
(51) Int. Cl.: A61F 2/38, A61F 2/00, A61F 2/30

(54) **Composant fémoral d'une prothèse unicompartimentale de genou**
Femurkomponente einer unikondylären Knieprothese
Femoral component for a unicompartmental knee prosthesis

(30) Priorité: 20.05.2010 FR 1053913
(43) Date de publication de la demande: 23.11.2011
(73) Titulaire: Tornier, 38330 Montbonnot-Saint-Martin (FR)
(72) Inventeur: Julita, Olivier, 38800, LE PONT DE CLAIX (FR); Cassagne, Frédéric, 38330, MONTBONNOT SAINT MARTIN (FR); Badet, Roger, 69003, LYON (FR); De Foort, Koen, 6522 VJ, NIJMEGEN (NL); Deschamps, Gérard, 71640, GIVRY (FR); Donell, Simon, NORWICH, NR4 7UY (GB); Bruderer, James, 71220, LE ROUSSET (FR); Roumazeille, Bertrand, 59800, LILLE (FR)
(74) Mandataire: Grand, Guillaume

(56) Documents cités:
- FR-A1- 2 758 715
- FR-A1- 2 836 821
- FR-A1- 2 908 040
- US-A1- 2006 235 537

## Description

La présente invention concerne une prothèse unicompartimentale de genou, et plus particulièrement le composant fémoral d'une prothèse unicompartimentale de genou cimentée.

Les prothèses unicompartimentales de genou cimentées sont implantées au niveau de l'un des compartiments fémoro-tibiaux du genou, soit interne, soit externe. La mise en place de ce genre de prothèse, appelée arthroplastie unicompartimentale, est destinée à remplacer uniquement l'un des deux compartiments du genou, interne ou externe, suivant la localisation des dommages, quelle qu'en soit l'origine (liés à l'arthrose, post-traumatiques, nécrose...), latéralisés, du côté du fémur et du côté du tibia, en utilisant un ciment osseux chirurgical.

Une prothèse unicompartimentale du genou est généralement constituée d'un composant fémoral interne et/ou externe coopérant avec un composant tibial interne et/ou externe correspondant.

Une prothèse unicompartimentale de genou peut être une prothèse dite à coupe ou une prothèse dite de resurfaçage, selon que le fémur nécessite ou non une coupe distale. La présente invention s'applique tant aux prothèses dites à coupe qu'aux prothèses dites de resurfaçage.

Le profil de la face externe convexe du composant fémoral de la prothèse est tel qu'il permet une cinématique du genou prothésé proche de la cinématique originelle du genou. Le profil de la face interne du composant fémoral de la prothèse est critique pour le positionnement et la fixation du composant fémoral au fémur. En effet, après la pose d'un composant fémoral d'une prothèse unicompartimentale de genou, celui-ci est soumis à des contraintes mécaniques importantes, lesquelles sollicitent également la fixation du composant sur l'os, durant les différentes phases de fonctionnement : marche, accroupissement, etc. A ce jour, le descellement aseptique est l'une des principales causes d'échec des prothèses unicompartimentales de genou.

Ainsi, il est essentiel que le composant fémoral d'une prothèse soit positionné avec précision sur l'os qui nécessite une réparation, puis immobilisé de manière rigide et durable.

De manière courante, on effectue une préparation osseuse correspondant à la forme interne du composant fémoral. Par la suite, on interpose du ciment qui, pour une efficacité maximale, doit être pressurisé lors de l'implantation du composant fémoral afin de lier ce dernier au condyle fémoral lors de la prise du ciment. Par ailleurs, le positionnement est effectué à l'aide d'un plot d'ancrage osseux solidaire de la prothèse et inséré dans l'os du fémur, et par le contact entre les surfaces correspondantes osseuses et prothétiques. Ce positionnement peut également être effectué au moyen d'un élément en saillie présentant la forme d'un rail, d'une croix, etc.

FR-A-2 758 715 propose un composant fémoral de prothèse unicompartimentale, dans laquelle l'axe du plot d'ancrage et la surface d'appui postérieure de la prothèse sont parallèles dans le plan sagittal. Toutefois, une telle prothèse n'est pas entièrement satisfaisante, puisque durant son implantation sur l'os, le ciment qui est initialement appliqué sur les surfaces distale et postérieure du composant fémoral n'est pas uniformément pressé contre les coupes d'os distale et postérieure. En effet, lorsque que le ciment est pressé par la surface distale contre la coupe d'os distale, une prise du ciment optimale avec l'os est créée ; tandis qu'au niveau de la surface postérieure, l'alignement entre la surface postérieure du composant fémoral et la coupe d'os postérieure induit un raclage du ciment, ce qui empêche sa présence en quantité suffisante et sa compression entre la face postérieure du composant fémoral et la coupe d'os postérieure.

US-A-2006/235537 décrit un composant fémoral d'une prothèse unicompartimentale de genou, comprenant une face externe et une face interne. La face interne comporte une surface distale et une surface postérieure reliée par une surface de chanfrein. Les surfaces distale et postérieure sont chacune munie d'un plot d'ancrage osseux et sont adaptées pour être appuyées contre des surfaces osseuses fémorales correspondantes avec interposition d'un ciment. La surface d'appui postérieure est tournée vers la surface d'appui distale, avec une inclinaison d'environ 83°. L'axe central du plot d'ancrage osseux équipant la surface distale est incliné par rapport au plan de la surface d'appui postérieure d'environ 25°, plus généralement entre 20° et 30°. Une telle prothèse n'est pas entièrement satisfaisante, notamment en termes d'implantation et de comportement mécanique.

FR 2908040 divulgue les caractéristiques énoncées dans le préambule de la revendication indépendante.

Le but de la présente invention est de prévoir un composant fémoral de prothèse permettant un positionnement correct sur l'os réséqué, ainsi qu'un ancrage et une prise du ciment améliorés, en particulier pour éviter le relâchement sous une contrainte de flexion importante.

A cet effet, l'invention a pour objet un composant fémoral d'une prothèse unicompartimentale de genou, tel que défini à la revendication 1.

Ainsi l'invention permet d'améliorer à la fois les étapes d'implantation du composant fémoral et de prise du ciment, de même que le comportement mécanique du composant fémoral de son côté postérieur, d'une part en créant une compression du ciment sur la surface osseuse postérieure au moyen d'un mouvement de translation divergent, et d'autre part en augmentant la résistance à la contrainte de cisaillement lors de la flexion du genou. En effet, il est essentiel que la fixation du composant fémoral d'une prothèse unicompartimentale de genou soit optimale sur l'os pour supporter les efforts d'expulsion en flexion qui sollicitent le ciment en cisaillement au niveau postérieur. L'invention permet un positionnement correct sur l'os réséqué, une limitation des efforts d'expulsion en flexion ainsi qu'une prise du ciment améliorée sur la face postérieure afin de garantir que le composant fémoral ne se descelle pas.

D'autres caractéristiques avantageuses de l'invention, prises isolément ou en combinaison, sont spécifiées aux revendications dépendantes 2 à 11.

Est également décrite ici une méthode chirurgicale de mise en place d'un composant fémoral telle que défini ci-dessus, cette méthode comprenant :
- une étape de préparation de l'os du fémur au niveau d'un condyle, pour réaliser au moins une coupe postérieure ;
- une étape de préparation de la face interne du composant fémoral à l'aide d'un ciment osseux chirurgical ;
- une étape d'implantation du composant fémoral selon la direction de l'axe central du plot d'ancrage osseux, dans laquelle les surfaces d'appui distale et postérieure se rapprochent progressivement des surfaces d'os respectivement distale et postérieure selon un mouvement de translation divergent, en créant une contrainte de compression du ciment sur l'os du fémur ; et
- une étape de prise du ciment.

En outre, l'étape de préparation de l'os du fémur au niveau d'un condyle selon cette méthode peut comporter une coupe distale. Cette méthode peut également inclure une étape de pose de ciment sur la ou les coupes osseuses. De plus, cette méthode peut inclure une étape de finition et/ou de contrôle et/ou d'équilibrage et/ou de test.

L'invention sera mieux comprise à la lecture de la description qui va suivre, donnée uniquement à titre d'exemple non limitatif et faite en se référant aux dessins sur lesquels :
- la figure 1 est une vue en élévation d'un composant fémoral d'une prothèse unicompartimentale conforme à l'invention,
- les figures 2 et 3 sont des vues en élévation selon respectivement les flèches Il et III de la figure 1,
- la figure 4 est une coupe partielle selon la ligne IV-IV de la figure 2,
- la figure 5 est une coupe partielle selon la ligne V-V de la figure 2, et
- les figures 6, 7 et 8 sont des vues en élévation analogues à la figure 1, qui illustrent des étapes successives de l'implantation du composant fémoral sur un os du fémur.

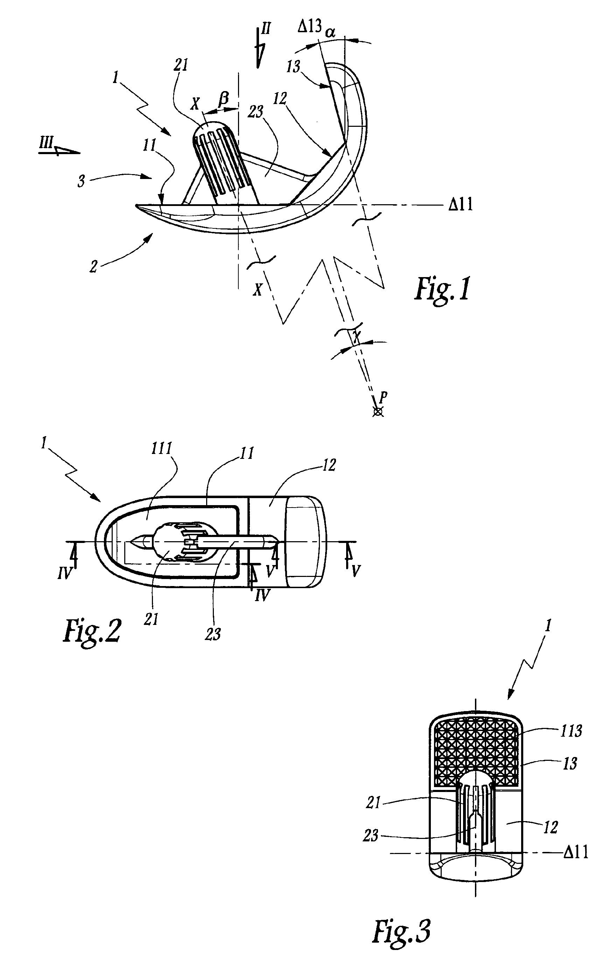

Sur les figures 1 à 5 est représenté un composant fémoral d'une prothèse unicompartimentale 1, qui présente une forme générale de coupelle, avec une face externe convexe 2 et une face interne 3 de profil sensiblement concave.

La face externe convexe 2 du composant fémoral 1 reproduit sensiblement l'anatomie d'un condyle fémoral externe ou interne, car elle est destinée à reproduire la cinématique articulaire du genou en coopérant avec le composant tibial.

La face interne 3 du composant fémoral 1 comprend trois surfaces planes 11, 12, 13 distinctes, qui sont aptes à venir en appui contre des coupes correspondantes d'un condyle fémoral. Selon les conventions anatomiques, les surfaces 11 et 13 peuvent être qualifiées respectivement de surfaces d'appui distale et postérieure, tandis que la surface 12 forme un chanfrein intermédiaire reliant les surfaces 11 et 13. Dans ce cas, le composant 1 appartient à une prothèse dite à coupe, pour laquelle le condyle fémoral F nécessite une coupe distale. En outre, les surfaces planes distales 11 et postérieures 13 définissent respectivement les plans associés Δ11 et Δ13, visibles sur la figure 1.

La normale au plan Δ11 de la surface 11 forme avec le plan Δ13 de la surface 13 un angle α non nul, qui dans l'exemple représenté est égal à 15°. A titre de variante non représentée, cet angle α est choisi entre 10° et 20°. Ainsi, les surfaces 11 et 13 ne s'étendent pas dans des plans perpendiculaires l'un à l'autre, mais forment entre elles un angle aigu du côté de la face interne 3, comme bien visible sur la figure 1. Autrement dit, la surface 13 est tournée vers la surface 11 et le plan associé Δ11, du côté de la face interne 3.

Un plot d'ancrage osseux 21 sensiblement cylindrique est disposé sur la surface distale 11. Son axe central X-X est incliné d'un angle β non nul par rapport à la normale au plan Δ11 de la surface 11, de manière qu'en parcourant axialement le plot 21 depuis son extrémité liée à la surface 11 jusqu'à son extrémité libre, on s'éloigne progressivement des surfaces 12 et 13. Dans l'exemple représenté, l'angle β vaut 20°. A titre de variante non représentée, cet angle β peut être choisi entre 15° et 25°, ou de préférence entre 15° et 20°. Dans tous les cas, l'angle α est choisi strictement inférieur à l'angle β : de la sorte, l'axe X-X est incliné par rapport au plan Δ13 de la surface 13, et le point qui est référencé P sur la figure 1, défini par leur intersection, est situé du côté de la face externe 2. Eu égard aux valeurs des angles α et β envisagées plus haut, l'angle γ non nul et strictement positif, formé entre l'axe X-X et la surface 13, est inférieur ou égal à 15 °.

Le plot d'ancrage osseux 21 délimite une pluralité de rainures longitudinales 22, réparties sur sa circonférence. Une nervure de rigidité 23 s'étend depuis le plot 21 dans un plan qui est perpendiculaire à la surface 11 et qui comprend l'axe X-X. La nervure de rigidité 23 s'étend, à sa base, sur la surface 11 et sur la surface 12.

Comme visible sur les figures 2 et 3, le composant fémoral 1 est ici symétrique par rapport au plan de la nervure 23. A titre de variante non représentée, le composant fémoral peut être asymétrique.

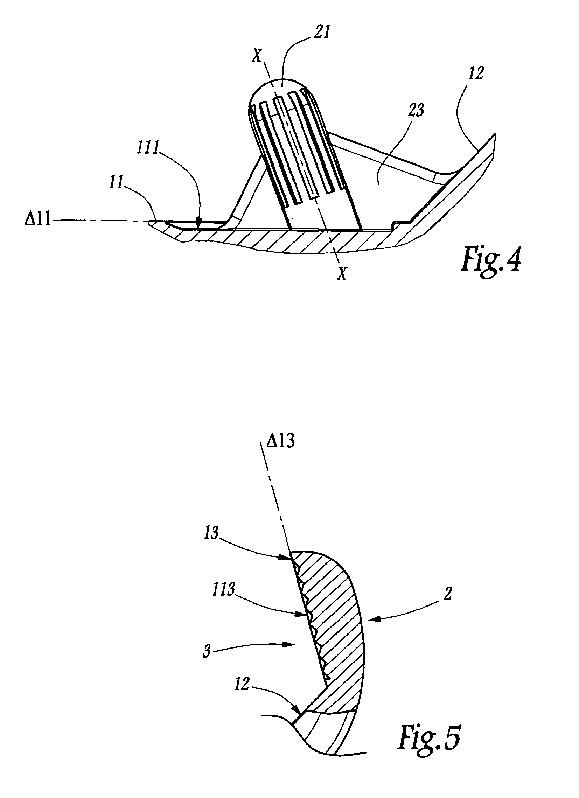

Comme visible sur les figures 2 et 4, la surface 11 délimite une cavité de réception de ciment 111, configurée en cuvette dont le fond est plat dans l'exemple considéré. Comme visible sur les figures 3 et 5, la surface d'appui postérieure 13 délimite elle aussi une cavité de réception de ciment 113, présentant un relief pyramidal.

En pratique, la profondeur et l'étendue du relief pyramidal peuvent varier pour obtenir une rugosité de la cavité 113 plus ou moins importante. Grâce à ses creux et protubérances, ce relief permet d'obtenir des volumes de ciment améliorant la résistance au cisaillement de la surface correspondante. De plus, lors de l'impaction du composant fémoral, le relief va permettre d'aider le ciment à pénétrer dans l'os spongieux pour un meilleur ancrage. A titre de variantes non représentées, d'autres configurations en relief peuvent être mises en oeuvre, par exemple des rainures.

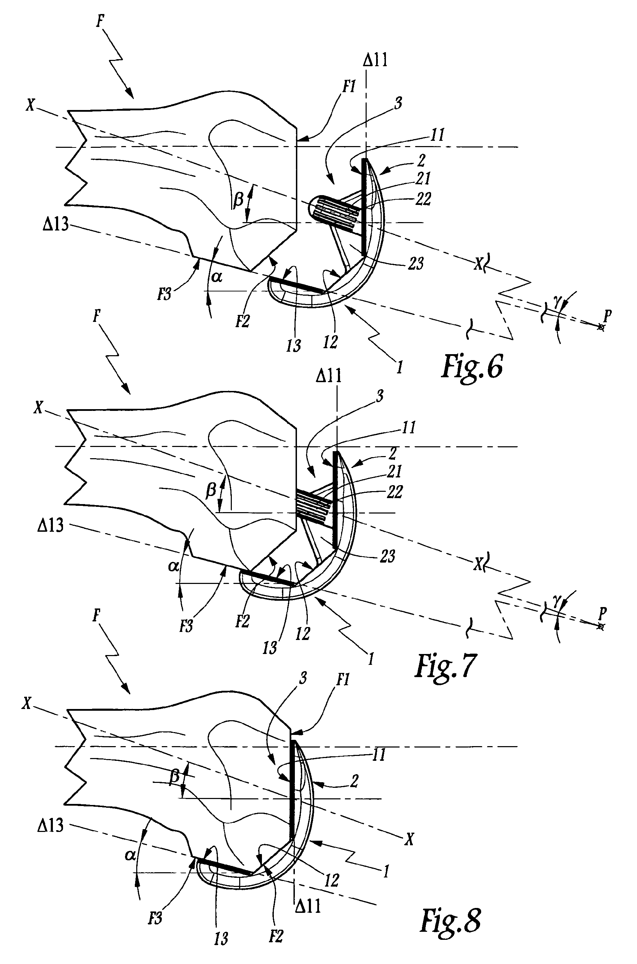

La mise en place du composant fémoral 1 sur un os du fémur F va maintenant être décrite en regard des figures 6 à 8.

La figure 6 montre le composant fémoral 1 avant son implantation sur l'os du fémur F, plus précisément dans le condyle fémoral. Un ciment osseux chirurgical, indiqué par des traits en surépaisseur, est déposé sur les surfaces 11 et 13, en comblant l'espace libre des cavités 111 et 113.

La figure 7 montre le composant fémoral 1 pendant son implantation dans le condyle fémoral F. L'implantation est réalisée selon la direction de l'axe X-X du plot 21, du côté de la face interne 3 du composant fémoral 1, afin que les surfaces d'appui 11, 12 et 13 se rapprochent progressivement de coupes d'os fémorales correspondantes F1, F2 et F3, préalablement réalisées. La présence de l'angle γ formé entre l'axe X-X du plot 21 et le plan Δ13 de la surface 13 permet d'effectuer un mouvement de translation divergent pour la surface 13, par rapport au mouvement de translation de la surface 11. Ainsi le ciment présent sur la surface 13 est progressivement compressé contre la coupe d'os postérieure F3, sans être raclé.

Le plot 21 et la nervure 23 permettent de faciliter le positionnement et l'implantation du composant fémoral 1 par le chirurgien.

La figure 8 représente le composant fémoral 1 lorsqu'il est implanté dans le condyle fémoral F. Le ciment est bien réparti entre la face interne 3 du composant fémoral 1 et le condyle fémoral F, notamment au niveau de la surface d'appui postérieure 13. En effet, on comprend que les cavités de réception de ciment 111 et 113 permettent d'optimiser la quantité de ciment chirurgical utilisée lors de l'implantation du composant fémoral 1, ainsi que la résistance de la liaison sous certaines contraintes : la cavité 111, configurée en cuvette à fond plat, permet d'obtenir une épaisseur de ciment optimale pour la résistance à la contrainte de compression au détriment de la résistance à la contrainte de cisaillement ; tandis que la cavité 113, avec son relief rugueux, permet d'obtenir une meilleure résistance au cisaillement au détriment de l'épaisseur de ciment.

Ces deux types de cavités de réception de ciment étant combinés ici, on obtient une bonne résistance à la contrainte de compression durant l'extension, en même temps qu'une bonne résistance à la contrainte de cisaillement durant la flexion du genou prothésé.

Selon un mode de réalisation non représenté, la surface distale 11 qui appartient à la face interne 3 du composant 1 et qui est apte à venir en appui contre une surface correspondante d'un condyle fémoral F, n'est pas plane. En effet, lorsque le composant 1 mis en oeuvre appartient à une prothèse dite de resurfaçage, pour laquelle le condyle fémoral F ne nécessite pas de coupe distale, alors la surface distale 11 peut présenter une forme courbe, correspondant à la surface complémentaire resurfacée de l'os du fémur F.

Dans ce cas, un plan virtuel associé à la surface distale 11 peut être défini par la ligne périphérique de la surface distale 11. Ce plan virtuel associé est comparable au plan Δ11, visible sur la figure 1. Ainsi, l'angle α est défini comme étant l'angle entre la normale au plan virtuel associé à la surface 11 et le plan Δ13 de la surface postérieure 13. La surface 13 est tournée vers la surface 11 et son plan virtuel associé, du côté de la face interne 3.

Ainsi, le composant fémoral 1 selon l'invention permet d'améliorer le positionnement, l'ancrage et la prise du ciment sur le condyle fémoral F, de même que le comportement du composant fémoral 1 lorsqu'il est soumis à de fortes contraintes de cisaillement en flexion.

## Revendications

1. Composant fémoral (1) d'une prothèse unicompartimentale de genou, comprenant une face externe convexe (2) et une face interne (3), laquelle comporte une surface distale (11) munie d'un plot d'ancrage osseux (21) et une surface postérieure substantiellement plane (13), ces surfaces distale et postérieure (11, 13) étant adaptées pour être appuyées contre des surfaces osseuses fémorales correspondantes (F1, F3) avec interposition d'un ciment, l'axe central (X-X) du plot d'ancrage osseux (21) étant incliné par rapport au plan (Δ13) de la surface d'appui postérieure (13), le point (P) défini par leur intersection étant situé du côté de la face externe (2), **caractérisé en ce que** la surface d'appui postérieure (13) est tournée vers la surface d'appui distale (11) et **en ce que** l'axe central (X-X) du plot d'ancrage osseux (21) et le plan de la surface d'appui postérieure (13) forment un angle (γ) non nul et inférieur ou égal à 15°.

2. Composant fémoral selon la revendication 1, **caractérisé en ce que** la surface distale (11) est substantiellement plane et adaptée pour être appuyée contre une coupe fémorale correspondante (F1) avec interposition d'un ciment.

3. Composant fémoral selon l'une des revendications précédentes, **caractérisé en ce que** la normale au plan (Δ11) associé à la surface d'appui distale (11) forme un premier angle (α) avec le plan (Δ13) de la surface d'appui postérieure (13) et un second angle (β) avec l'axe central (X-X) du plot d'ancrage osseux (21), les premier et second angles (α, β) étant différents l'un de l'autre, le premier angle (α) étant inférieur au second angle (β).

4. Composant fémoral selon la revendication 3, **caractérisé en ce que** le premier angle (α) est compris entre 10 et 20 ° et le second angle (β) est compris entre 15 et 25 °.

5. Composant fémoral selon l'une des revendications précédentes, caractérisé en ce la normale au plan (Δ11) associé à la surface d'appui distale (11) forme un angle (β) avec l'axe central (X-X) du plot d'ancrage osseux (21) qui est égal à 20°.

6. Composant fémoral selon l'une des revendications précédentes, **caractérisé en ce que** les surfaces d'appui distale (11) et postérieure (13) délimitent chacune une cavité de réception de ciment (111, 113).

7. Composant fémoral selon la revendication 5, **caractérisé en ce que** la cavité de réception de ciment (113) de la surface d'appui postérieure (13) présente une rugosité plus importante que celle de la cavité de réception de ciment (111) de la surface d'appui distale (11).

8. Composant fémoral selon l'une des revendications 6 ou 7, **caractérisé en ce que** la cavité de réception de ciment (113) de la surface d'appui postérieure (13) présente un relief incluant au moins une pyramide et/ou au moins une rainure.

9. Composant fémoral selon l'une des revendications 6, 7 ou 8, **caractérisé en ce que** la cavité de réception de ciment (111) de la surface d'appui distale (11) est en forme de cuvette.

10. Composant fémoral selon l'une des revendications précédentes, **caractérisé en ce que** le plot d'ancrage osseux (21) est sensiblement cylindrique et délimite au moins une rainure longitudinale (22).

11. Composant fémoral selon l'une des revendications précédentes, **caractérisé en ce qu'**une nervure de rigidité (23) s'étend depuis le plot d'ancrage osseux (21) dans un plan qui est perpendiculaire à la surface d'appui distale (11) et qui comprend l'axe central (X-X) du plot d'ancrage osseux (21).

## Claims

1. Femoral component (1) of a unicompartmental knee prosthesis, comprising a convex outer surface (2) and an inner surface (3) which comprises a distal surface (11) provided with a bone anchorage pin (21) and a substantially flat rear surface (13), these distal and rear surfaces (11, 13) being designed to be supported against corresponding femoral bone surfaces (F1, F3) with interposition of a cement, the central axis (X-X) of the bone anchorage pin (21) being inclined relative to the plane (Δ13) of the rear support surface (13), the point (P) defined by their intersection being situated on the outer surface side (2), **characterised in that** the rear support surface (13) faces towards the distal support surface (11), and **in that** the central axis (X-X) of the bone anchorage pin (21) and the plane of the rear support surface (13) form an angle (γ) which is not zero and is equal to 15° or less.

2. Femoral component according to claim 1, **characterised in that** the distal surface (11) is substantially flat, and is designed to be supported against a corresponding femoral cross-section (F1) with interposition of a cement.

3. Femoral component according to one of the preceding claims, **characterised in that** the line perpendicular to the plane (Δ11) which is associated with the distal support surface (11) forms a first angle (α) with the plane (Δ13) of the rear support surface (13), and a second angle (β) with the central axis (X-X) of the bone anchorage pin (21), the first and second angles (α, β) being different from one another, the first angle (α) being smaller than the second angle (β).

4. Femoral component according to claim 3, **characterised in that** the first angle (α) is between 10° and 20°, and the second angle (β) is between 15° and 25°.

5. Femoral component according to one of the preceding claims, **characterised in that** the line perpendicular to the plane (Δ11) which is associated with the distal support surface (11) forms an angle (β) with the central axis (X-X) of the bone anchorage pin (21) which is equal to 20°.

6. Femoral component according to one of the preceding claims, **characterised in that** the distal (11) and rear (13) support surfaces each delimit a cavity (111, 113) for receipt of cement.

7. Femoral component according to claim 5, **characterised in that** the cavity (113) for receipt of cement of the rear support surface (13) has a roughness which is greater than that of the cavity (111) for receipt of cement of the distal support surface (11).

8. Femoral component according to one of claims 6 or 7, **characterised in that** the cavity (113) for receipt of cement of the rear support surface (13) has a relief which includes at least one pyramid and/or at least one groove.

9. Femoral component according to one of claims 6, 7 or 8, **characterised in that** the cavity (111) for receipt of cement of the distal support surface (11) is in the form of a bowl.

10. Femoral component according to one of the preceding claims, **characterised in that** the bone anchorage pin (21) is substantially cylindrical, and delimits at least one longitudinal groove (22).

11. Femoral component according to one of the preceding claims, **characterised in that** a strengthening rib (23) extends from the bone anchorage pin (21) on a plane which is perpendicular to the distal support surface (11), and comprises the central axis (X-X) of the bone anchorage pin (21).

## Patentansprüche

1. Femurkomponente (1) einer unikondylären Knieprothese, die eine konvexe Außenfläche (2) und eine Innenfläche (3) umfasst, wobei letztere eine mit einer Knochenverankerungsstütze (21) versehene distale Fläche (11) und eine im Wesentlichen ebene hintere Fläche (13) aufweist, wobei diese distale Fläche (11) und diese hintere Fläche (13) angepasst sind, sich unter Einfügung eines Zements gegen korrespondierende femorale Knochenflächen (F1, F3) abzustützen, wobei die Mittelachse (X-X) der Knochenverankerungsstütze (21) in Bezug auf die Ebene (Δ13) der hinteren Abstützfläche (13) geneigt ist und der Punkt (P), der durch ihren Schnittpunkt definiert ist, auf der Seite der Außenfläche (2) liegt, **dadurch gekennzeichnet, dass** die hintere Abstützfläche (13) zur distalen Abstützfläche (11) gerichtet ist und dass die Mittelachse (X-X) der Knochenverankerungsstütze (21) und die Ebene der hinteren Abstützfläche (13) einen Winkel (γ) bilden, der nicht null ist und kleiner oder gleich 15° ist.

2. Femurkomponente nach Anspruch 1, **dadurch gekennzeichnet, dass** die distale Fläche (11) im Wesentlichen eben ist und angepasst ist, um sich unter Einfügung von Zement gegen eine korrespondierende femorale Schnittfläche (F1) abzustützen.

3. Femurkomponente nach einem der vorhergehenden Ansprüchen, **dadurch gekennzeichnet, dass** die Normale zur Ebene (Δ11), die der distalen Abstützfläche (11) zugeordnet ist, einen ersten Winkel (α) mit der Ebene (Δ13) der hinteren Abstützfläche (13) und einen zweiten Winkel (β) mit der Mittelachse (X-X) der Knochenverankerungsstütze (21) bildet, wobei der erste und zweite Winkel (α, β) unterschiedlich zueinander sind und der erste Winkel (α) kleiner als der zweite Winkel (β) ist.

4. Femurkomponente nach Anspruch 3, **dadurch gekennzeichnet, dass** der erste Winkel (α) zwischen 10° und 20° liegt und der zweite Winkel (β) zwischen 15 und 25° liegt.

5. Femurkomponente nach einem der vorhergehenden Ansprüche, **dadurch gekennzeichnet, dass** die Normale zur Ebene (Δ11), die der distalen Abstützfläche (11) zugeordnet ist, einen Winkel (β) mit der Mittelachse (X-X) der Knochenverankerungsstütze (21) bildet, der gleich 20° ist.

6. Femurkomponente nach einem der vorhergehenden Ansprüche, **dadurch gekennzeichnet, dass** die distale und hintere Abstützfläche (11, 13) jeweils einen Hohlraum (111, 113) zur Aufnahme von Zement begrenzen.

7. Femurkomponente nach Anspruch 5, **dadurch gekennzeichnet, dass** der Hohlraum (113) zur Aufnahme von Zement der hinteren Abstützfläche (13) eine größere Rauigkeit als der Hohlraum (111) zur Aufnahme von Zement der distalen Abstützfläche (11) aufweist.

8. Femurkomponente nach einem der Ansprüche 6 oder 7, **dadurch gekennzeichnet, dass** der Hohlraum (113) zur Aufnahme von Zement der hinteren Abstützfläche (13) ein Relief aufweist, das mindestens eine Pyramide und/oder mindestens eine Nut einschließt.

9. Femurkomponente nach einem der Ansprüche 6 oder 7, **dadurch gekennzeichnet, dass** der Hohlraum (111) zur Aufnahme von Zement der distalen Abstützfläche (11) in Form einer Küvette ausgebildet ist.

10. Femurkomponente nach einem der vorhergehenden Ansprüche, **dadurch gekennzeichnet, dass** die Knochenverankerungsstütze (21) im Wesentlichen zylindrisch ist und mindestens eine Längsnut (22) begrenzt.

11. Femurkomponente nach einem der vorhergehenden Ansprüche, **dadurch gekennzeichnet, dass** eine Versteifungsrippe (23) sich von der Knochenverankerungsstütze (21) in einer Ebene erstreckt, die senkrecht zur distalen Abstützfläche (11) liegt und die die Mittelachse (X-X) der Knochenverankerungsstütze (21) einschließt.
